# EUROPEAN PATENT SPECIFICATION

(11) **EP 1 033 971 B1**
(45) Date of publication and mention of the grant of the patent: **09.09.2009**
(21) Application number: 98958388.5
(22) Date of filing: 01.12.1998
(51) Int. Cl.: A61K 9/127, A61K 38/19

(54) **COMBINED CHEMO-IMMUNOTHERAPY WITH LIPOSOMAL DRUGS AND CYTOKINES**
KOMBINIERTE CHEMO-IMMUNOTHERAPIE MIT LIPOSOMALEN ARZNEISTOFFEN UND CYTOKINEN
TRAITEMENT CHIMIO ET IMMUNOTHERAPEUTIQUE COMBINE A L'AIDE DE MEDICAMENTS ET DE CYTOKINES ENCAPSULES DANS DES LIPOSOMES

(30) Priority: 04.12.1997 US 67697 P
(43) Date of publication of application: 13.09.2000
(73) Proprietor: Yissum Research Development Company of the Hebrew University of Jerusalem Ltd, Jerusalem 91042 (IL); HADASIT MEDICAL RESEARCH SERVICES AND DEVELOPMENT LTD., 91120 Jerusalem (IL)
(72) Inventor: GABIZON, Alberto, A., 96920 Jerusalem (IL); KEDAR, Eliezer, 96920 Jerusalem (IL); BARENHOLZ, Yechezkel, 93707 Jerusalem (IL)
(74) Representative: Vossius & Partner
(86) International application number: PCT/IL1998/000586
(87) International publication number: WO 1999/027908

(56) References cited:
- EP-A- 0 433 765
- EP-A- 0 546 951
- WO-A-85/00968
- WO-A-90/13293
- WO-A-91/05545
- WO-A-91/05546
- DE-A- 4 430 593
- DATABASE WPI Week 8814 Derwent Publications Ltd., London, GB; AN 88-096332 XP002102116 & JP 63 048 225 A (GREEN CROSS CORP) , 29 February 1988

## Description

The present invention relates to a composition for antitumor therapy, and more particularly to a combination therapy using a chemotherapeutic drug and an immunostimulating cytokine. Sequential administration of these two components, whereby the immunostimulating cytokine is encapsulated in multi-lamellar liposomes, is shown to have a significant antitumor effect as compared to administration of the individual components, in free form or in liposomes.

### References

Adler, A. et al., Cancer Biotherapy 10:293-306 (1995).
Curran, D.P. et al., Angew. Chem. Intl. Ed. Eng. 34(23/24):2683-4 (1996).
Gabizon, A. et al., Adv. Drug Delivery Reviews 24(2-3):337-344 (1997).
Kedar, E. et al., J. Immunotherapy 16:47-59 (1994).
Lasic, D. and Martin, F., Eds., STEALTH LIPOSOMES CRC Press, Boca Raton, FL (1995).
Papahadjopoulos, D. et al., Proc. Natl. Acad. Sci. USA 88:11460-11464 (1991).
Sears, B.D., U.S. Pat. No. 4,426,330 (1984).
Sears, B.D., U.S. Pat. No. 4,534,899 (1985a).
Szoka, F., Jr. et al., U.S. Pat. No. 4,235,871 (1980b).
Szoka, F., Jr. et al., Ann. Re. Biophys. Bioeng. 9:467 (1980).
Tirosh, O. et al., J. Chem. Soc. Perk. Trans. II 2:383-389 (1997).
Woodle, M.C. et al., U.S. Pat. No. 5,013,556 (1991).

Despite prolific research in the area of cancer chemotherapy, such treatment remains far from satisfactory. The inability of chemotherapeutic drugs to reach the tumor site, intrinsic and acquired cross-resistance to multiple chemotherapeutic agents, and, especially, the high toxicity of many of these agents all contribute to treatment failures.

The use of immunostimulating cytokines, such as IL-2 and interferon-α, has proven to be effective in treatment of a proportion of patients with malignancies such as melanoma and renal cell carcinoma, both alone and in combination with other therapeutic agents. However, major problems limit their wide clinical use, including rapid plasma clearance, biodistribution to nonrelevant tissues, and high toxicity. Furthermore, their efficacy has been low in treatment of the most common tumors, *e.g*. colorectal, mammary, prostate, and lung carcinomas.

The present invention includes, in one aspect, a composition for use in antitumor therapy, which comprises a combination of a chemotherapeutic drug and an immunostimulating cytokine, whereby the immunostimulating cytokine is encapsulated in multi-lamellar vesicles.

Administration of the combination produces a greater therapeutic effect than a combination of the effects produced by the liposome-encapsulated components administered individually.

The invention also includes a composition for antitumor therapy in which a chemotherapeutic drug, encapsulated in liposomes, is administered in combination with a cytokine, which is also encapsulated in liposomes, namely multi-lamellar liposomes. Thereby, the drug is encapsulated in liposomes which contain about 1-10 mole percent of a lipid having a polar head group derivatized with a polyethylene glycol (PEG) chain which has a molecular weight of between 750 and 10,000 daltons. The therapeutic effect of this combination is greater than a combination of the effects produced by the liposome-encapsulated drug and the cytokine administered individually.

In all cases, administration of the cytokine preferably follows administration of the liposome-encapsulated drug.

The chemotherapeutic drug is preferably selected from cis-platin, a chemotherapeutic anthraquinone, and a topoisomerase I inhibitor, such as camptothecin or a camptothecin analog. More preferably, the drug is adriamycin (doxorubicin), in which case the liposome-encapsulated form of the drug is preferably DOXIL®.

The immunostimulating cytokine is preferably selected from the group consisting of interleukin-2 (IL-2), IL-12, IL-15, IL-18, IFN-γ, IFN-α, IFN-β, TNF-α, G-CSF, and GM-CSF. More preferably, the cytokine is IL-2.

The encapsulating liposomes employed in the composition and method preferably contain at least one lipid selected from dimyristoyl phosphatidyl choline (DMPC), dimyristoyl phosphatidyl glycerol (DMPG), 1,2-distearoyl-3-trimethylammonium propane (DSTAP), phosphatidyl choline, phosphatidyl ethanolamine, and cholesterol.

The liposomes may be small unilamellar vesicles (SUV), defined as having a mean diameter of approximately 20 to 100 nm, or large unilamellar vesicles (LUV), defined as having a mean diameter of approximately 100 to 200 nm. Such liposomes preferably contain about 1-10 mole percent of a lipid having a polar head group derivatized with a polyethylene glycol (PEG) chain which has a molecular weight of between 750 and 10,000 daltons.

Alternatively, the liposomes may be large multilamellar vesicles (MLV) having a mean diameter of approximately 250 to 2000 nm. The MLV may also contain a PEG-derivatized lipid as described above. -

In preferred embodiments, the chemotherapeutic drug is encapsulated in vesicles having a mean diameter of approximately 50 to 120 nm, and containing about 1-10 mole percent of a lipid having a polar head group derivatized with a polyethylene glycol (PEG) chain as described above. In another preferred embodiment, the cytokine is encapsulated in liposomes containing dimyristoyl phosphatidyl choline (DMPC) plus 0 to 50 mole percent of at least one lipid selected from dimyristoyl phosphatidyl glycerol (DMPG) and 1,2-distearoyl-3-trimethylammonium propane (DSTAP).

These and other objects and features of the invention will become more fully apparent when the following detailed description of the invention is read in conjunction with the accompanying drawings.

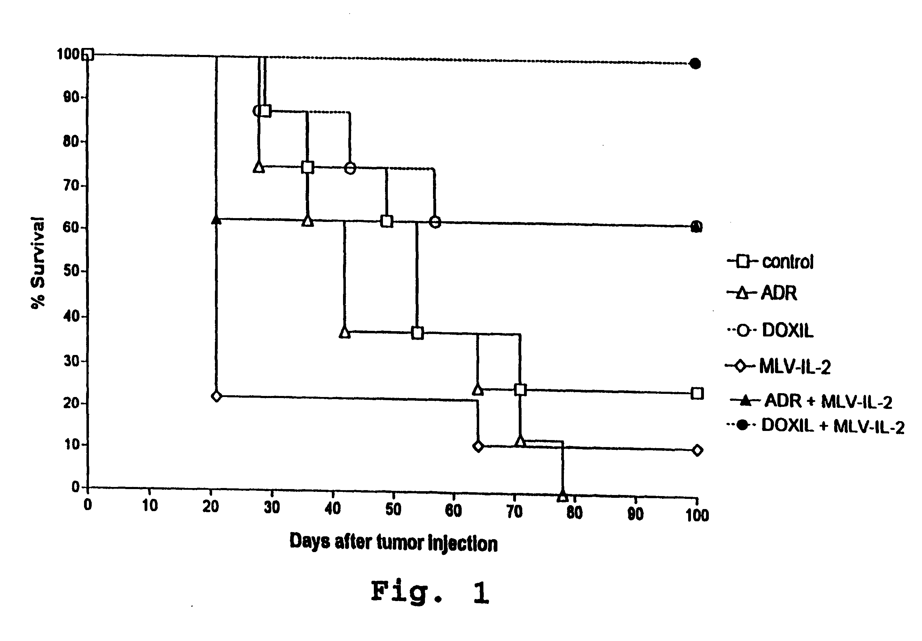

Figure 1 shows the survival rate of BALB/c mice injected intraperitoneally with 5 × 10⁵ M109 tumor cells (lung adenocarcinoma) and subsequently treated with free adriamycin or DOXIL®, respectively, alone or in combination with intraperitoneal IL-2 in DMPC/DMPG MLV liposomes, or with liposomal IL-2 alone; and

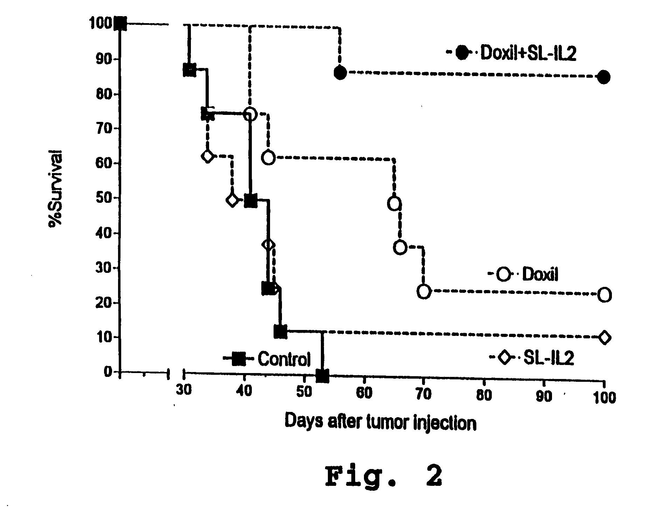

Figure 2 shows the survival rate of BALB/c mice injected intravenously with 5 × 10⁵ M109 tumor cells and subsequently treated with DOXIL® (at day 7), alone or in combination with intravenous IL-2 in Stealth® PEGylated SUV liposomes (at days 11, 14, and 17), or with liposomal IL-2 alone (at days 11, 14, and 17).

### I. Definitions

The terms below have the following meanings unless indicated otherwise.

"Vesicle-forming lipids" refers to amphipathic lipids which have hydrophobic and polar head group moieties, and which (a) can form bilayer vesicles in water, as exemplified by phospholipids, or (b) can be stably incorporated into lipid bilayers, with the hydrophobic moiety in contact with the interior, hydrophobic region of the bilayer membrane, and the polar head group mciety oriented toward the exterior, polar surface of the membrane.

The vesicle-forming lipids of this type typically include one or two hydrophobic acyl hydrocarbon chains or a steroid group, and may contain a chemically reactive group, such as an amine, acid, ester, aldehyde or alcohol, at the polar head group. Included in this class are the phospholipids, where the two hydrocarbon chains are typically between about 14-22 carbon atoms in length, and have varying degrees of unsaturation. Representative examples are phosphatidyl choline (PC), phosphatidyl ethanolamine (PE), phosphatidic acid (PA), phosphatidyl inositol (PI), sphingomyelin (SM), negatively charged lipids such as dimyristoyl phosphatidyl glycerol (DMPG), and positively charged lipids such as 1,2-distearoyl-3-trimethylammonium propane (DSTAP). The liposomes may also contain sterols, such as cholesterol, which do not form liposomes themselves but can be incorporated into, and may stabilize, liposomes containing lipids such as those described above.

A "Cetus unit" (CU) is equal to six International Units (IU) of Immunological Activity, the international reference standard of a biological preparation of interleukin-2 (IL-2). The term "unit" used herein in reference to cytokine levels refers to Cetus units.

### II. Liposomal Compositions

### A. Lipid Components

Various vesicle-forming lipids, as defined above, may be used in the present liposomal compositions, according to methods well known in the art. Preferred lipids for the current invention allow long-term storage of the liposome-entrapped agents and effective release of these components upon administration. Representative lipids include, but are not limited to, dimyristoyl phosphatidylcholine (DMPC), dimyristoyl phosphatidylglycerol (DMPG), cholesterol, egg phosphatidylcholine (egg PC), phosphatidyl ethanolamine (PE), distearoyl phosphatidyl ethanolamine (DSPE), phosphatidyl inositol (PI), 1,2-distearoyl-3-trimethylammonium propane (DSTAP), 1,2-dimyristoyl-3-trimethylammonium propane (DMTAP), and combinations thereof.

The vesicle-forming lipids, preferably those making up SUV's, may contain about 1-10 mole percent of a lipid having a polar head group, typically a phosphate containing head group, derivatized with a polyethylene glycol (PEG) chain which has a molecular weight of between 750 and 10,000 daltons. The rate of clearance of liposomes from circulation is typically reduced by employing such PEG-derivatized, or "PEGylated", lipids. PEG coating is believed to inhibit nonspecific adsorption of serum proteins, thereby preventing nonspecific recognition of liposomes by macrophages (Papahadjopoulos, *et al.,* 1991). Another advantage of these long-circulating liposomes is their good extravasation capacity and high accumulation in tumors (Lasic and Martin, 1995; Gabizon, *et al.,* 1997). They are also referred to as sterically stabilized liposomes, SSL; or Stealth® liposomes.

The preparation of such lipids is described in, for example, Woodle, *et al.,* 1991; Sears (1984, 1985); Tirosh *et al*. (1997) or copending and co-owned application having U.S. serial number 08/570,440. The PEG chain may be linked directly to the phosphatidic acid head group of a phospholipid. Various other linkages are possible; for example, lipids containing a phosphatidyl ethanolamine (PE) or other amino head group may be conveniently coupled to activated PEG chains via reaction with brominated PEG. PEG-modified lipids are also commercially available, *e*.*g.* from Sequus Corporation, Menlo Park, CA.

### B. Preparation of Liposomes and Liposomal Compositions

Liposomes may be prepared by a variety of techniques, such as those detailed in Szoka *et al.* (1980b). To form multilamellar vesicles (MLV's), a mixture of vesicle- forming lipids dissolved in a suitable solvent is evaporated in a vessel to form a thin film, which is then hydrated by an aqueous medium to form MLV's, typically with sizes between about 0.1 to 10 microns. Tert-butanol is a preferred solvent for the process. The MLV's may then be downsized to a desired size range by extruding the aqueous suspension through a polycarbonate membrane having a selected uniform pore size, typically 0.05 to 1.0 microns.

Preparations of MLV's or REV's (described below) may be treated, *e.g*. by extrusion, sonication or high pressure homogenization, to produce unilamellar vesicles. Small unilamellar vesicles (SUV's) are characterized by sizes in the 30-100 nm range, while large unilamellar vesicles (LUV's) are defined as those having mean diameters of about 100-200 nm. SUV's may also be formed directly by high pressure homogenization of an aqueous dispersion of lipids.

Various methods are available for encapsulating other agents in liposomes. Preparation of SSL-encapsulated IL-2 is described in Kedar *et al.* (1994). In this procedure, generally, the lipid components, including a PEG-substituted lipid, are dissolved in t-butanol. The solution is sonicated, and IL-2 is added with further sonication. The mixture is lyophilized and rehydrated, forming MLV's, which can then be downsized by high pressure homogenization or by successive extrusion through polycarbonate filters. These downsizing methods gave vesicles having diameters of 50-80nm and about 200nm, respectively. The procedure achieved approximately 80-90 % encapsulation of the IL-2.

In the reverse phase evaporation method (Szuka, *et al.,* 1980a) a nonaqueous solution of vesicle-forming lipids is dispersed with a smaller volume of an aqueous medium to form a water-in-oil emulsion. The agent to be incorporated is included either in the lipid solution, in the case of a lipophilic agent, or in the aqueous medium, in the case of a water-soluble agent. After removal of the lipid solvent, the resulting gel is converted to liposomes. These reverse phase evaporation vesicles (REVs) have typical average sizes between about 0.2-4 microns and are predominantly oligolamellar, that is, containing one or a few lipid bilayer shells. The REVs may be sized by extrusion, if desired, to give oligolamellar vesicles having a maximum selected size between about 0.05 to 1.5 microns.

Other methods for adding additional components to liposomal compositions include colyophilization with other components and redispersion of the resulting solid to form MLV's. In a method described by Adler, *et al*. (1995), an aqueous solution of the agent to be encapsulated is added to a t-butanol solution of lipids. The mixture is sonicated and lyophilized, and the resulting powder is rehydrated.

Liposome compositions containing an entrapped agent may be treated after final sizing, if necessary, to remove free (non-entrapped) agent. Conventional separation techniques, such as centrifugation, diafiltration, and molecular-sieve chromatography are suitable for this purpose. The composition may also be sterilized by filtration through a conventional 0.22 or 0.45 micron depth filter.

To form the compositions of the current invention, the concentration of drug and/or cytokine in the liposomes is preferably effective to give a protein/lipid weight ratio between about 1:100 and 1:1000.

Stabilizers may also be added to the liposomal compositions. For example, addition of a metal chelator such as Desferal^{™} or diethylenetriamine pentaacetic acid (DTPA) to the lyophilization medium, at a concentration of 100 µM, has been shown to reduce activity loss of entrapped IL-2 during liposome preparation and storage at 4°C. Antioxidants such as BHT or Vitamin E may also be included.

For long term storage, the compositions may be stored as the dry lyophilized powder, which is stable for at least a year at 4°C, and hydrated to form an aqueous suspension before use.

### III. Combined Chemotherapy/Cytokine Therapy

### A. Formulations

Cytokines useful for enhancing antitumor activity of chemotherapeutic drugs include IL-2, IL-12, IL-15, IL-18, IFN-γ, IFN-α, IFN-β, TNF-α, G-CSF, and GM-CSF. A preferred cytokine for the present invention is IL-2 (interleukin 2), which acts as a growth and maturation factor for T-lymphocytes.

In the present invention, multilamellar liposomes are used for encapsulation of the cytokine. Other types of liposomal formulations include LUV or SUV, as defined above, as well as OLV (oligolamellar vesicles) and MW (multivesicular vesicles), composed of vesicle-forming lipids such as those described above. These types are, however, not included within the present invention. Combinations of lipids are generally most effective (see, for example, Kedar *et al.,* 1994). One type of formulation employs SUV or LUV, having a mean diameter of approximately 50 to 120 nm, containing about 1-10 mole percent of a lipid having a polar head group derivatized with a polyethylene glycol (PEG) chain (also referred to as a PEGylated lipid). Formulation A below is one example. Preferred formulations employ dimyristoyl phosphatidyl choline (DMPC) and, optionally, up to 50 mole percent of at least one lipid selected from dimyristoyl phosphatidyl glycerol (DMPG) and 1,2-distearoyl-3-trimethylammonium propane (DSTAP). In these formulations, the proportion of DMPG and/or DSTAP is more preferably 5 - 25 mole percent. Formulation B below is one example. In all cases, small quantities (up to about one mole percent) of stabilizers such as tocopherol or Desferal^{™} may be included.

For the experiments described below, liposomal IL-2 was prepared in two formulations, using IL-2 obtained from Chiron Corporation (Emeryville, CA), according to known methods such as those described above. Formulation A employed sterically stabilized (SSL) small unilamellar vesicles (SUV) composed of ²⁰⁰⁰PEG-DSPE (N-carbamyl-(polyethylene glycol methyl ether)-1,2-distearoyl-sn-glycero-3-phosphoethanolamine triethylammonium salt, provided by Sequus Corporation), egg phosphatidyl choline, and cholesterol in a molar ratio of about 5:55:40. The vesicles were about 50-70 nm in diameter. Encapsulation efficiency of IL-2 was greater than 80 % , based on an *in vitro* IL-2 bioassay (*i.e.*, > 80 % of the initial amount of added IL-2 became encapsulated in liposomes).

Formulation B employed multilamellar vesicles (MLV) composed of DMPC-DMPG (dimyristoyl phosphatidyl choline - dimyristoyl phosphatidyl glycerol) in a 9:1 molar ratio. The vesicles were approximately 500-1500 nm in size, and the encapsulation efficiency was approximately > 90%. This high efficiency of encapsulation was achieved at a lipid:IL-2 ratio (wt:wt) of 1000:1 for DMPC alone, and 100:1 for DMPC containing DMPG or DSTAP.

The chemotherapeutic drug is preferably encapsulated in liposomes having about 1-10 mole percent of a PEGylated lipid, as described above. For example, DOXIL®, a stable formulation of adriamycin in Stealth® liposomes, is available from SEQUUS Pharmaceuticals, Inc. (Menlo Park, CA). Free adriamycin is available, *e.g.*, from Cetus Oncology Corp. (Emeryville, CA) as a formulation of doxorubicin hydrochloride and lactose.

Other chemotherapeutic drugs which are also preferred for the present method include other anthraquinones, such as epirubicin, daunorubicin, and mitoxanthrone, and cis-platin. Also contemplated are topoisomerase I inhibitors such as camptothecin and its analogs, *e*.*g.* topotecan and irinotecan, also designated CPT-11. Camptothecin is isolated from the stem wood of the Chinese tree *Camptotheca aciminata*; preparation of the above noted analogs has been described by, *e.g.*, Curran *et al.* (1996).

### B. Liposomal Adriamycin - Liposomal IL-2

The effect of adriamycin, used alone or in combination with interleukin-2 (IL-2), where each component was in free or liposome-encapsulated form, on the survival rate of BALB/c mice infected with tumor cells, was tested as described below.

B1. Lung Adenocarcinoma Model: IL-2 in MLV. Six groups of BALB/c mice were injected intraperitoneally with 5 × 10⁵ M109 tumor cells (day 0). Free adriamycin or DOXIL®, respectively, were administered intravenously on day 7 at a dose of 8 mg/kg, and intraperitoneal cytokine treatment was initiated 3 days later. Liposomal IL-2 (formulation B; MLV DMPC/DMPG (9:1 mole ratio) liposomes containing IL-2) was given once daily (50,000 CU/mouse) on days 10, 13 and 16. Control groups received no treatment or received the IL-2 treatment alone.

Each group, consisting of 8-9 mice, was inspected for survival up to 100 days after tumor inoculation. Table I shows the number of survivors at the end of the experiment and the median survival time obtained; Figure 1 shows the survival curves for all groups.

**TABLE I**

| **GROUP** | **TREATMENT** | **NUMBER OF SURVIVING MICE/TOTAL** | **MEDIAN SURVIVAL (DAYS)** |
|---|---|---|---|
| 1 | Control | 2/8 | 54 |
| 2 | ADR | 0/8 | 42 |
| 3 | ADR + MLV-IL-2 | 5/8 | > 100 |
| 4 | DOXIL^{®} | 5/8 | >100 |
| 5 | DOXIL^{®} + MLV-IL-2 | 8/8 | > 100 |
| 6 | MLV-IL-2 | 1/9 | 21 |

As Table I shows, adriamycin (ADR) in combination with MLV-IL-2 (liposomal IL-2, formulation B) was much more effective than either adriamycin alone or liposomal IL-2 alone, both of which showed lower survival rates than the control. When liposomal adriamycin (DOXIL®) was administered alone, or when non-liposomal adriamycin was combined with liposomal IL-2, five of eight mice survived for the duration of the test.

The best result, *i*.*e*. survival of all subjects for 100 days or more, was observed for the combination of liposomal ADR (DOXIL®) with liposomal IL-2. In terms of number of surviving subjects, the effect of the combination treatment was greater than a combination of the effects of the individual treatments.

B2. Metastatic Lung Adenocarcinoma Model: IL-2 in MLV (Formulation B) and PEG-Derivatized SUV (SSL). In this experiment, BALB/c mice were injected intravenously with 5 × 10⁵ M109 tumor cells (day 0). Free adriamycin or DOXIL®, respectively, were administered intravenously on day 7 (8 mg/kg), followed 3 days later by intravenous cytokine treatment. Liposomal IL-2 (Formulation A; PEGylated SUV containing IL-2) was given once daily (50,000 CU/mouse) on days 11, 14 and 17. Control groups received no treatment or received the IL-2 treatment alone.

Each group, consisting of 8-9 mice, was inspected for survival up to 100 days after tumor inoculation. Results are shown in Table II and Figure 2.

**TABLE II**

| **GROUP** | **TREATMENT** | **NUMBER OF SURVIVING MICE/TOTAL** | **MEDIAN SURVIVAL (DAYS)** |
|---|---|---|---|
| 1 | Control | 0/8 | 43 |
| 2 | ADR | 2/8 | 56 |
| 3 | DOXIL^{®} | 1/8 | 66 |
| 4 | SSL-IL-2 | 0/8 | 41 |
| 5 | DOXIL^{®} + SSL-IL-2 | 7/9 | > 100 |

As a comparison of groups 3-5 shows, the combined treatment with DOXIL® and liposomal IL-2 was significantly more effective than treatment with either liposomal component alone, particularly in terms of the number of subjects surviving for the duration of the test, *i.e.* 100 days or more (7 out of 9 compared to 0-1 out of 8). In this aspect, the combined treatment was significantly more effective than a combination of the effects derived from the individual therapies.

In a second, more extensive study, nine groups of BALB/c mice were injected intraperitoneally with 5 × 10⁵ M109 tumor cells. Free adriamycin or DOXIL® (8 mg/kg) were administered intraperitoneally 7 days later, followed 3 days later by intravenous cytokine treatment. The cytokine, given once daily (50,000 CU/mouse) on days 10, 13 and 16, consisted of free IL-2, IL-2 in Formulation A (Stealth® PEGylated SUV), or IL-2 in Formulation B (9:1 molar DMPC/DMPG MLV).

Each group, consisting of 8 mice, and an untreated control group of 11 mice, were inspected for survival up to 120 days after tumor inoculation. Results are shown in Table III.

**TABLE III**

| **GROUP** | **TREATMENT** | **NUMBER OF TUMOR FREE MICE/TOTAL** | **MEDIAN SURVIVAL (DAYS)** |
|---|---|---|---|
| 1 | Control | 3/11 | 69 |
| 2 | ADR | 4/8 | 100 |
| 3 | ADR/free IL-2 | 3/8 | 53 |
| 4 | ADR/MLV-IL-2 | 4/8 | 92 |
| 5 | ADR/SSL-IL-2 | 3/8 | 72 |
| 6 | DOXIL® | 4/8 | 102 |
| 7 | DOXIL®/free IL-2 | 5/8 | > 120 |
| 8 | DOXIL®/MLV-IL-2 | 7/8 | > 120 |
| 9 | DOXIL®/SSL-IL-2 | 5/8 | > 120 |

In this study, administration of free ADR and IL-2 showed little or no benefit over free ADR alone (groups 2-5). However, combinations of either free or liposomal IL-2 with the chemotherapeutic drug in liposomes (DOXIL®) showed clear benefits over administration of the drug alone (groups 6-9). Overall, the groups (8 and 9) treated with a combination of both components in liposomes showed superior results. Group 8, in particular, showed a high survival rate and almost a complete absence of tumors.

B3. Subcutaneous colon carcinoma model: IL-2 in MLV. In this test, 7 groups of BALB/c mice were injected in the footpad with 10⁵ C26 colon carcinoma cells. Seven days later, 8 mg/kg free or liposomal adriamycin was administered i.v. Free or liposomal IL-2, as shown in Table IV, was administered i.p. according to the schedule described above. Results are shown in Table IV.

**TABLE IV**

| **GROUP** | **TREATMENT** | **NUMBER OF TUMOR FREE MICE, DAY 30** | **NUMBER OF TUMOR FREE MICE, DAY 65** |
|---|---|---|---|
| 1 | Control | 0/7 | 0/7 |
| 2 | ADR | 0/7 | 0/7 |
| 3 | ADR/free IL-2 | 0/7 | 0/7 |
| 4 | ADR/MLV-IL-2 | 1/8 | 0/8 |
| 5 | DOXIL® | 4/8 | 102 |
| 6 | DOXIL®/free IL-2 | 3/8 | 2/8 |
| 7 | DOXIL®/MLV-IL-2 | 6/8 | 4/8 |

As the data shows, administration of liposomal drug alone was somewhat beneficial, but only the group receiving the combined liposomal treatment showed significant recovery from tumors. In this group (group 7), it was also observed that the tumors were significantly smaller than in the other groups.

### IV. Administration

For use in humans, a therapeutically effective dose of the composition typically corresponds to 20-100 mg adriamycin/m² of body surface. For IL-2, a preferred dose corresponds to 50,000 - 500,000 CU per square meter of body surface. Administration may be by intraperitoneal (ip), subcutaneous (sc), intravenous (iv), intraarterial (ia), or intramuscular (im) injection. Liposomes in the form of large multilamellar vesicles (MLV's) are preferred for intraperitoneal, subcutaneous or intramuscular administration, while SUV's are preferred for intravenous as well as intramuscular administration.

As shown above, administration of liposome-encapsulated chemotherapeutic drug is followed by administration of the liposome-encapsulated cytokine. While specific time intervals and courses of treatment have been shown in the examples above, it is understood that dosages, time intervals between courses, and the number of courses of treatment, for both drug and cytokine, may be varied depending on the extent of symptoms and the condition of the patient.

## Claims

1. A pharmaceutical composition for use in an antitumor therapy comprising an immunostimulating cytokine encapsulated in multi-lamellar liposomes (MLV) and a chemotherapeutic drug as a combined preparation for sequential administration.

2. An immunostimulating cytokine encapsulated in multi-lamellar liposomes (MLV) for use in anti-tumor treatment of a tumor patient in combination with a chemotherapeutic drug treatment.

3. The pharmaceutical composition according to claim 1 or the cytokine according to claim 2, wherein said immunostimulating cytokine is selected from interleukin-2 (IL-2), IL-12, IL-15, IL-18, IFN-γ, IFN-α, IFN-β, TNF-α, G-CSF, and GM-CSF.

4. The pharmaceutical composition according to claim 1 or 3 or the cytokine according to claim 2 or 3, wherein the cytokine is encapsulated in liposomes comprising at least one lipid selected from the group consisting of dimyristoyl phosphatidyl choline (DMPC), dimyristoyl phosphatidyl glycerol (DMPG), 1,2-distearoyl trimethylammonium propane (DSTAP), phosphatidyl choline, phosphatidyl ethanolamine, and cholesterol.

5. The pharmaceutical composition or the cytokine according to claim 4, wherein the cytokine is encapsulated in liposomes comprising dimyristoyl phosphatidyl choline (DMPC) in combination with 0-50 mole percent of at least one lipid selected from dimyristoyl phosphatidyl glycerol (DMPG) and 1,2-distearoyl trimethylammonium propane (DSTAP).

6. The pharmaceutical composition or the cytokine according to claim 5, wherein said liposome is composed of DMPC and DMPG in a molar ratio of about 9:1.

7. The pharmaceutical composition of any one of claims 1 or 3 to 6 or the cytokine according to any one of claims 2 to 6, wherein said chemotherapeutic drug is selected from the group consisting of anthraquinones and cis-platin.

8. The pharmaceutical composition or the cytokine according to claim 7, wherein said anthraquinone is selected from doxorubicin, epirubicin, daunorubicin and mitoxanthrone.

9. The pharmaceutical composition or the cytokine according to any one of the preceding claims, wherein said chemotherapeutic drug is encapsulated in a liposome.

10. The pharmaceutical composition or the cytokine according to claim 9, wherein said chemotherapeutic drug is a polyethylene glycol coated liposomal doxorubicin, wherein said polyethylene glycol has a molecular weight of between 750 and 10,000 daltons.

## Patentansprüche

1. Arzneimittel zur Verwendung in einer Antitumortherapie, das ein immunstimulatorisches Cytokin, das in multi-lamellaren Liposomen (MLV) verkapselt ist, und einen chemotherapeutischen Arzneistoff als kombiniertes Präparat für die sequenzielle Gabe umfasst.

2. Immunstimulatorisches, in multi-lamellare Liposomen (MLV) verkapseltes Cytokin zur Verwendung bei der Antitumorbehandlung eines Tumorpatienten in Kombination mit einer Behandlung mit einem chemotherapeutischen Arzneistoff.

3. Arzneimittel nach Anspruch 1 oder Cytokin nach Anspruch 2, wobei das immunstimulatorische Cytokin aus Interleukin-2 (IL-2), IL-12, IL-15, IL-18, IFN-γ, IFNa, IFN-β, TNF-α, G-CSF und GM-CSF ausgewählt ist.

4. Arzneimittel nach Anspruch 1 oder 3 oder Cytokin nach Anspruch 2 oder 3, wobei das Cytokin in Liposomen verkapselt ist, umfassend mindestens ein Lipid ausgewählt aus der Gruppe bestehend aus Dimyristoylphosphatidylcholin (DMPC), Dimyristoylphosphatidylglycerol (DMPG), 1,2-Distearoyltrimethylammoniumpropan (DSTAP), Phosphatidylcholin, Phosphatidylethanolamin und Cholesterol.

5. Arzneimittel oder Cytokin nach Anspruch 4, wobei das Cytokin in Liposomen verkapselt ist, die Dimyristoylphosphatidylcholin (DPMC) in Kombination mit 0 bis 50 Molprozent mindestens eines Lipids umfassen, das aus Dimyristoylphosphatidylglycerol (DMPG) und 1,2-Distearoyltrimethylammoniumpropan (DSTAP) ausgewählt ist.

6. Arzneimittel oder Cytokin nach Anspruch 5, wobei sich das Liposom aus DMPC und DMPG in einem Molverhältnis von etwa 9:1 zusammensetzt.

7. Arzneimittel nach einem der Ansprüche 1 oder 3 bis 6 oder Cytokin nach einem der Ansprüche 2 bis 6, wobei der chemotherapeutische Arzneistoff aus Anthrachinonen und cis-Platin ausgewählt ist.

8. Arzneimittel oder Cytokin nach Anspruch 7, wobei das Anthrachinon aus Doxorubicin, Epirubicin, Daunorubicin und Mitoxanthron ausgewählt ist.

9. Arzneimittel oder Cytokin nach einem der vorherigen Ansprüche, wobei der chemotherapeutische Arzneistoff in einem Liposom verkapselt ist.

10. Arzneimittel oder Cytokin nach Anspruch 9, wobei der chemotherapeutische Arzneistoff ein Polyethylenglykol-beschichtetes liposomales Doxorubicin ist, wobei das Polyethylenglykol ein Molekulargewicht zwischen 750 und 10.000 Dalton aufweist.

## Revendications

1. Composition pharmaceutique pour utilisation dans une thérapie anti-tumeur comprenant une cytokine immunostimulante encapsulée dans des liposomes multi-lamellaires (MLV) et un médicament chimiothérapeutique comme préparation combinée pour l'administration séquentielle.

2. Cytokine immunostimulante encapsulée dans des liposomes multi-lamellaires (MLV) pour utilisation dans un traitement anti-tumeur d'un patient atteint de tumeur en combinaison avec un traitement à base de médicament chimiothérapeutique.

3. Composition pharmaceutique selon la revendication 1 ou la cytokine selon la revendication 2, où ladite cytokine immunostimulante est sélectionnée parmi interleukine-2 (IL-2), IL-12, IL-15, IL-18, IFN-γ, IFN-α, IFN-β, TNF-α, G-CSF et GM-CSF.

4. Composition pharmaceutique selon la revendication 1 ou 3 ou la cytokine selon la revendication 2 ou 3, où la cytokine est encapsulée dans des liposomes contenant au moins un lipide sélectionné dans le groupe consistant en dimyristoyle phosphatidyle choline (DMPC), dimyristoyle phosphatidyle glycérol (DMPG), 1,2-distéaroyle triméthylammonium propane (DSTAP), phosphatidyle choline, phosphatidyle éthanolamine et cholestérol.

5. Composition pharmaceutique ou la cytokine selon la revendication 4, où la cytokine est encapsulée dans des liposomes comprenant dimyristoyle phosphatidyle choline (DMPC) en combinaison avec 0-50 pour cent en mole d'au moins un lipide sélectionné parmi dimyristoyle phosphatidyle glycérol (DMPG) et 1,2-distéaroyle triméthylammonium propane (DSTAP).

6. Composition pharmaceutique ou la cytokine selon la revendication 5, où ledit liposome est constitué de DMPC et DMPG en un rapport molaire d'environ 9:1.

7. Composition pharmaceutique selon l'une quelconque des revendications 1 ou 3 à 6 ou la cytokine selon l'une quelconque des revendications 2 à 6, où ledit médicament chimiothérapeutique est sélectionné dans le groupe consistant en anthraquinones et cis-platine.

8. Composition pharmaceutique ou la cytokine selon la revendication 7, où ladite anthraquinone est sélectionnée parmi doxorubicine, épirubicine, daunorubicine et mitoxanthrone.

9. Composition pharmaceutique ou la cytokine selon l'une quelconque des revendications précédentes, où ledit médicament chimiothérapeutique est encapsulé dans un liposome.

10. Composition pharmaceutique ou la cytokine selon la revendication 9, où ledit médicament chimiothérapeutique est un polyéthylène glycol revêtu de doxorubicine liposomale, où ledit polyéthylène glycol a un poids moléculaire entre 750 et 10 000 daltons.
